# FASCICULE DE BREVET EUROPEEN

(11) **EP 1 518 124 B1**
(45) Date de publication et mention de la délivrance du brevet: **14.01.2009**
(21) Numéro de dépôt: 03762723.9
(22) Date de dépôt: 02.07.2003
(51) Int. Cl.: G01N 33/569, G01N 33/96

(54) **METHODE DE DIAGNOSTIC SEROLOGIQUE**
VERFAHREN ZUR SEROLOGISCHEN DIAGNOSE
SEROLOGICAL DIAGNOSTIC METHOD

(30) Priorité: 03.07.2002 FR 0208324
(43) Date de publication de la demande: 30.03.2005
(73) Titulaire: Université de la Méditerranée, Aix-Marseille II, 13284 Marseille Cedex 07 (FR)
(72) Inventeur: RAOULT, Didier, 13008 Marseille (FR)
(74) Mandataire: Domange, Maxime
(86) Numéro de dépôt international: PCT/FR2003/002050
(87) Numéro de publication internationale: WO 2004/005932

(56) Documents cités:
- US-A- 4 757 002
- GARROTE J A ET AL: "A NOVEL VISUAL IMMUNOASSAY FOR COALIAC DISEASE SCREENING" EUROPEAN JOURNAL OF CLINICAL INVESTIGATION, BLACKWELL SCIENTIFIC PUBLICATIONS, XX, vol. 29, no. 8, août 1999 (1999-08), pages 697-699, XP001097615 ISSN: 0014-2972

## Description

La présente invention concerne une méthode de diagnostic des maladies infectieuses humaines. Plus particulièrement, elle concerne le domaine du diagnostic indirect par sérologie. Une des méthodes de laboratoire pour le diagnostic des maladies infectieuses humaines est la sérologie microbienne. Il s'agit d'une méthode de diagnostic indirect qui repose sur la recherche dans le sérum du patient d'anticorps spécifiques de l'agent microbien infectieux, à savoir une bactérie, un virus, un parasite ou un champignon responsable d'une pathologie (désignés ci-dessous "microbe"). Cette méthode de diagnostic est primordiale pour le diagnostic des infections à microorganisme de culture et d'identification difficile, en particulier les virus et les bactéries intracellulaires strictes et les bactéries intracellulaires facultatives des genres *Rickettsie, Coxiella, Bartonella, Tropheryma, Ehrlichia, Chlamydia, Mycoplasma, Treponema, Boffelia*, et *Leptospira* par exemple.

Techniquement, la sérologie microbienne consiste à détecter dans le sérum du patient une réaction antigène - anticorps dans laquelle l'antigène est représenté par tout ou fraction de l'agent microbien infectieux à détecter et l'anticorps est représenté par les immunoglobulines humaines spécifiques dudit agent microbien infectieux présentes dans le sérum du patient. Elle peut être quantifiée en testant successivement une série de dilutions croissantes de raison 2 ou de raison 10 du sérum du patient.

Classiquement, une méthode de diagnostic sérologique comporte plus particulièrement les étapes suivantes :
**1.** dépôt de l'antigène sur un support solide tel que des microbilles de latex, notamment dans la technique de détection par agglutination dans laquelle des microbilles de latex sont recouvertes par l'antigène microbien à tester, et sont agglutinées les unes aux autres par le sérum de patient présentant des anticorps spécifiques, cette agglutination étant visible à l'oeil nu ; ou une lame de verre, ou microplaque de filtration pour les techniques de détection par immunofluorescence ou par technique enzymatique, notamment du type ELISA "Enzyme Linked Immunosorbent Assay" ou une feuille de nitrocellulose ou de nylon pour les techniques de détection du type Westem-blot. Dans laquelle les antigènes microbiens séparés par électrophorèse puis transférés sur un support solide (feuille de nitrocellulose ou de nylon) réagissent séparément les uns des autres avec le sérum du patient.Ces techniques de détection sont bien connues de l'homme de l'art,
**2.** décomplémentation du sérum par chauffage à 56°C pendant 30 minutes,
**3.** mise en contact de l'antigène correspondant à l'agent microbien infectieux, avec le sérum du patient, puis incubation sous des conditions de temps, de température, d'hygrométrie, d'agitation mécanique et de force ionique du milieu permettant la réaction antigène - anticorps,
**4.** lavage précautionneux et extensif permettant d'éliminer le sérum du patient non-fixé au support solide, en excès
**5.** application d'un anticorps secondaire de détection qui est une immunoglobuline anti-immunoglobuline humaine, notamment une immunoglobuline de chèvre conjuguée à une substance fluorochrome, généralement de l'iso-thio-sulfate de fluoresceine ou une enzyme, généralement une peroxydase et incubation sous des conditions de temps, de température, d'hygrométrie, d'agitation mécanique et de force ionique du milieu permettant la réaction antigène - anticorps,
**6.** lavage précautionneux et extensif permettant d'éliminer l'immunoglobuline conjuguée, non fixée, en excès,
**7.** détection d'une réaction par lecture à l'aide d'un appareillage tel qu'un microscope à fluorescence pour la technique d'immunofluorescence indirecte, ou un lecteur de densité optique pour la technique de détection enzymatique du type ELISA. La lecture de la réaction Western-Blot se fait à l'oeil nu, ou après acquisition numérique du gel et analyse par un logiciel de densitométrie.

Chacune de ces étapes peut être une source d'erreurs de manipulation qui résultent en une absence de réaction antigène-anticorps et donc à une réaction faussement négative. Une erreur de manipulation aboutit donc éventuellement à interpréter comme négative une réaction qui est en réalité positive (faux-négatif). Une erreur fréquente dans la réalisation des tests sérologiques, notamment pour les tests sérologiques en batterie réalisés sur un grand nombre de sérums à tester, est due à des défauts dans l'introduction des sérums à tester, notamment par pipetage. Ces erreurs interviennent notamment dans les étapes qui impliquent le déplacement de l'échantillon à tester, notamment par pipetage, certains récipients, notamment contenant le support solide sur lequel est déposé l'antigène à détecter, peuvent ne pas être remplis par inadvertance avec le sérum humain à tester. Il est connu que le pipetage de sérum est entaché d'un risque d'erreurs de 1‰, liée à un problème purement technique par absence de pipetage par la pipette, ou à une erreur humaine par absence de pipetage par inadvertance.

Ces erreurs imposent l'introduction de contrôles dans la réalisation de la réaction. L'incorporation systématique au cours de chaque nouvelle manipulation d'un sérum témoin négatif c'est à dire ne contenant pas d'anticorps spécifiques de l'antigène à tester permet d'interpréter les réactions positives. De même, l'incorporation d'un sérum témoin positif, c'est à dire contenant l'anticorps spécifique de l'antigène testé à un titre connu permet de vérifier la qualité de l'antigène et de l'immunoglobuline conjuguée.

S'il n'existe aucun contrôle de ce que le sérum à tester a bien été introduit dans le test sérologique, et si par inadvertance, le sérum à tester n'est pas introduit dans le test sérologique, la réaction antigène bactérien-anticorps sérique n'existera certainement pas, et le test sera interprété, faussement, comme négatif (faux-négatif).

DE 1000322 décrit la mise en oeuvre d'anticorps anti immunoglobulines humaine, d'origine animale, à titre de contrôle la présence de sérum dans un échantillon à tester par Westernblot. L'article Europeen Journal of clinical investigation, 1999 (29,697-699) de JA GARROTE et col. décrit un immuno essai de détection visuelle impliquant la mise en oeuvre d'une substance de détection constituée d'un conjugué de protéine A-or colloïdal.

La protéine A est un polypeptide de 42 kDa, et est un constituant de la paroi des souches de *Staphylococcus aureus,* des protéines similaires mais différentes sont caractérisées à la surface des bactéries du genre *Streptococcus* [Langone J.J. Adv. Immunol. 1982, 32 : 157-251].

Il est connu qu'une fraction de la paroi de *Staphylococcus aureus* se lie aux immunoglobulines humaines et que la protéine A est le constituant de la paroi de *Staphylococcus aureus* qui se lie au sérum humain [Forsgren A. and J. Sjöquist. J. Immunol. 1966, 97:822-827], et plus particulièrement à l'extrémité Fc des immunoglobulines humaines. La protéine A dispose de quatre sites de liaison à l'extrémité Fc des immunoglobulines, dont deux sites de fixation mobilisables lors d'une même réaction antigène-anticorps. L'analyse cristalline de cette réaction a été réalisée [Deisenhofer J. Biochemistry 1981, 20 : 2361-2370]. L'affinité de la protéine A pour les différents sérums animaux a été étudiée [Kronvall G. Seal US, Finstad J., Williams RC Jr. J. Immunol. 1970 104: 140-147 ; Richman DD, Cleueland PH, Oxman MN, Johnson Km. J. Immunol. 1982 128: 2300-2305] et cette protéine A présente une affinité pour les sérums d'origine animale, notamment le cheval, les bovins, le cochon, le lapin, le cobaye, la souris ; à un moindre degré le hamster, le rat et le mouton. En revanche, le sérum de poussin et de chèvre ne réagissent pas avec la protéine A.

*Staphylococcus aureus* a déjà été introduit comme antigène pour la détection dans des tests sérologiques d'anticorps spécifiques [Ryding U, Esperson F, Sôderquist B, Christensson B. Diag. Microbiol. Infect. Dis. 2002, 42 : 9-15]. Les travaux publiés ont montré l'absence de spécificité de ces tests.

L'objet de la présente invention est l'introduction d'un contrôle d'introduction du sérum à tester au cours des réactions de sérologies bactériennes.

Les inventeurs ont découvert de façon fortuite lors d'un travail sérologique chez des patients présentant une endocardite, que les sérums de ces patients réagissaient tous contre *Staphylococcus aureus* et ils ont démontré, selon la présente invention, qu'il s'agissait là d'une propriété générale, à savoir que la réaction d'un sérum humain avec un antigène contenant la protéine A n'était pas altérée dans le cas de sérum humain de pathologie infectieuse, y compris les pathologies infectieuses modifiant le plus fortement le taux et la nature des immunoglobulines sécrétées telles que les maladies virales impliquant les virus V.I.H., C.M.V. ou encore Epstein-Barr. En effet, s'il était connu que la protéine A se liait à tout sérum humain normal, il n'était pas connu que cette propriété était conservée pour des sérums humains prélevés chez des patients présentant différentes pathologies infectieuses. Il n'était pas non plus connu que le *Staphylococcus aureus* pouvait remplir ce rôle de « porteur » de protéine A au cours des réactions de sérologie bactérienne.

La présente invention consiste donc, essentiellement, à introduire une bactérie *Staphylococcus aureus* à titre d'antigène de contrôle que l'échantillon à tester contient bien un sérum d'origine humaine et à détecter une réaction antigène-immunoglobuline humaine avec une substance spécifique.

Les inventeurs ont en effet découvert que dans la mesure où la protéine A réagit avec des immunoglobulines humaines de façon non spécifique, et ce même en cas de pathologie infectieuse importante, il est possible d'utiliser cette protéine A comme contrôle positif de l'introduction d'un sérum humain dans l'échantillon à tester, puisque d'autre part la protéine A ne réagit pas avec certains anticorps.

Plus précisément, la présente invention fournit une méthode de diagnostic sérologique in-vitro dans laquelle on détecte la présence d'anticorps spécifiques d'un agent microbien infectieux dans un échantillon à tester, caractérisée en ce que l'on contrôle que ledit échantillon à tester contient un sérum humain en détectant si des immunoglobulines humaines réagissent avec un antigène contenant une bactérie *Staphylococcus aureus.*

Selon l'invention, on réalise les étapes dans lesquelles :
- on fait réagir l'échantillon à tester avec un premier antigène (Ag₁) comprenant une bactérie entière *Staphylococcus aureus* comprenant la protéine A, et
- on détecte la présence d'un produit de réaction antigène-anticorps (Ag₁-Ac₁) dans lequel l'anticorps (Ac₁) est une immunoglobuline humaine, en faisant réagir ledit produit de réaction avec une substance de détection qui est une substance réagissant avec une immunoglobuline humaine et ne réagissant pas avec ledit premier antigène.

Par "détection d'immunoglobuline humaine réagissant avec un antigène contenant la protéine A", on entend que l'on est capable de déterminer un seuil de dilution de l'échantillon à tester contenant un sérum humain, et plus particulièrement, un seuil de dilution de sérum humain à tester, seuil au delà duquel on ne détecte plus ladite immunoglobuline humaine ayant réagit avec ledit antigène comprenant la protéine A compte tenu de la méthode de détection mise en oeuvre.

Selon l'invention, on réalise les étapes suivantes dans lesquelles :
a) on dépose sur un support solide ledit premier antigène comprenant une bactérie entière staphylococcus aureus contenant la protéine A (Ag₁) et un ou plusieurs autres seconds antigènes (Ag₂) qui sont caractéristiques d'un agent ou respectivement plusieurs agents infectieux microbiens et
b) on fait réagir les dits premiers (Ag₁) et seconds (Ag₂) antigènes avec un échantillon de sérum à tester, et
c) on détecte si une immunoglobuline humaine (Ac₁) réagit avec ledit premier antigène (Ag₁) en faisant réagir le produit de la réaction (Ag₁-Ac₁) avec un anticorps secondaire de détection (Ac₂) qui est une immunoglobuline anti-immunoglobuline humaine marquée qui ne réagit pas avec la protéine A.

Dans un mode de réalisation avantageux, ledit premier antigène est une bactérie entière *Staphylococcus aureus* comprenant la protéine A. On peut plus particulièrement utiliser les bactéries *Staphylococcus aureus* déposées dans les collections publiques telles que les bactéries déposées à l'A.T.C.C. sous le N°29213 et à la C.N.C.M. de l'Institut Pasteur (France) sous le numéro 65.8T.

Par ailleurs, en dehors des souches-types, toute souche bactérienne identifiée comme *Staphylococcus aureus* peut être utilisée comme antigéne Ag₁.

Avantageusement encore, on détecte la présence d'un dit produit de la réaction avec une immunoglobuline anti-immunoglobuline humaine qui est une immunoglobuline d'origine animale, de préférence, une immunoglobuline de chèvre ou de poussin.

Comme support solide, on peut utiliser tout dispositif adapté à la manipulation de suspensions cellulaires et bactériennes et notamment des tubes, des lames de verre, des tubes Bijoux ou des plaques rigides de microtitrage en polyéthylène, polystyrène, chlorure de polyvinyle ou nitrocellulose comportant des micropuits.

Comme type de marquage de l'immunoglobuline anti-humaine, on utilise donc avantageusement un marquage enzymatique, radioactif ou fluorescent, ce dernier type de marquage étant préféré.

L'expression « marquage fluorescent » signifie que l'anticorps a été rendu fluorescent par couplage ou complexation avec un agent fluorescent approprié tel que l'iso(thio)cyanate de fluorescéine.

L'expression « marquage radioactif » signifie que l'anticorps porte un isotope radioactif permettant de le doser par comptage de la radioactivité qui lui est associée, l'isotope pouvant être porté soit sur un élément de la structure de l'anticorps, par exemple les résidus de tyrosine constitutifs, soit sur un radical approprié qui lui a été fixé.

L'expression « marquage enzymatique » signifie que l'anticorps spécifique est couplé ou complexé à une enzyme qui, associée à l'emploi de réactifs appropriés, permet une mesure quantitative de cet anticorps spécifique.

Le substrat et les réactifs sont choisis de sorte que le produit final de la réaction ou de la séquence de réactions provoquée par l'enzyme et mettant en oeuvre ces substances soit :
- ou bien une substance colorée ou fluorescente qui diffuse dans le milieu liquide environnant l'échantillon testé et qui fait l'objet, soit de la mesure finale spectrophotométrique ou fluorimétrique, respectivement, soit d'une évaluation à l'oeil ; éventuellement par rapport à une gamme de teintes étalonnées,
- ou bien une substance colorée insoluble qui se dépose sur l'échantillon testé et qui peut faire l'objet, soit d'une mesure photométrique par réflexion, soit d'une évaluation à l'oeil, éventuellement par rapport à une gamme de teintes étalonnées.

Lorsque l'on utilise une immunoglobuline rendue fluorescente, la fluorescence associée à l'échantillon testé est lue directement sur un appareil approprié.

Lorsqu'on utilise une sonde radioactive, comme par exemple l'iode 125, la radioactivité associée à l'échantillon testé est complétée dans un compteur gamma selon toute modalité appropriée et par exemple après solubilisation des cellules par une solution alcaline (par exemple une solution de soude) et récupération de la solution contenant la radioactivité à l'aide d'un tampon absorbant.

Lorsque l'on utilise une enzyme sur l'anticorps spécifique, l'apparition d'un produit coloré ou fluorescent est obtenue en ajoutant une solution contenant le substrat de l'enzyme et un ou plusieurs réactifs auxiliaires permettant d'obtenir finalement comme produit de réaction, soit un produit coloré soluble dans le milieu, soit un produit coloré insoluble, soit un produit fluorescent soluble, comme cela a été expliqué précédemment. On mesure ensuite le signal lumineux provenant des échantillons ainsi traités, à l'aide de l'appareillage adapté à chaque cas : photomètre en transmission, ou en réflexion ou fluorimètre respectivement. Alternativement, on peut aussi évaluer à l'oeil la coloration obtenue, en s'aidant éventuellement d'une gamme de solutions colorées étalonnées.

En utilisant comme enzyme la phosphatase alcaline, le couplage de cette enzyme avec l'anticorps spécifique est effectué selon la méthode proposée par Boehringer Mannheim-Biochemica. Les substrats préférentiels de cette enzyme sont le paranitrophénylphosphate pour une lecture fluorométrique ou le bromo-5 chloro-4-umbelliféryl phosphate pour une lecture fluorométrique ou le bromo-5 chloro-4 indolyl-6 phosphate pour obtenir un produit de réaction coloré insoluble. On peut de même utiliser comme enzyme la β-galactosidase dont les substrats préférentiels sont l'orthonitrophényl β-D-galactropyranoside ou le méthyl-4 umbelliféryl β-D-galactopyranoside.

Préférentiellement, on peut coupler les anticorps spécifiques à la péroxydase. Dans ce cas ; le procédé de couplage est dérivé de celui décrit par M.B. Wilson et P.K. Nakane in Immunofluorescence and related staining techniques, W. Knapp, K. Kolubar, G. Wicks ed. Elsevier/North Holland. Amsterdam 1978, p. 215-224.

Les réactifs utilisés pour révéler la péroxydase conjuguée aux anticorps spécifiques contiennent de l'eau oxygénée, sustrat de l'enzyme, et un chromogène approprié par exemple de l'orthophénylénediamine ou l'acide azino-2-2' bis (éthyl-3 thiazoline sulfonique-6) ou ABTS pour obtenir un produit final de réaction coloré et soluble dans le milieu ou bien la diamino-3,3' benzidine ou l'amino-3 éthyl-9 carbazole ou le chloro-4 α-naphtol pour obtenir un produit final de réaction insoluble, ou bien l'acide parahydroxyphényl propionique pour obtenir un produit de réaction fluorescent soluble dans le milieu.

Un autre mode de réalisation de l'invention est l'utilisation d'immunoglobuline couplée à l'acétylcholinestérase.

L'acétylcholinestérase est couplée à l'anticorps en utilisant préférentiellement un procédé dérivé de celui décrit dans le brevet français n° 2 550 799 ou un procédé qui comporte schématiquement la préparation de fragments de l'anticorps par une technique connue, la modification de l'enzyme par réaction avec un agent hétérobifonctionnel approprié et enfin le couplage des produits ainsi obtenus. D'autres procédés connus de construction de conjugués immunoenzymatiques peuvent aussi être utilisés dans ce cas.

La révélation de l'activité enzymatique spécifiquement liée à l'antigène reconnu par le conjugué à l'acéthylcholinestérase est réalisée de préférence selon la technique bien connue qui emploie l'acéthylthiocholine comme substrat de l'enzyme et le réactif d'Ellman, ou acide dithio-5,5' nitro-2 benzoïque comme chromogène, selon toute variante adaptée au cas examiné, par exemple celle décrite par Pradelles et al., dans Anal. Chem. 1985, 57 :1170-1173.

Plus particulièrement encore, on réalise une série de tests à des dilutions de l'échantillon à tester croissantes et on met en oeuvre une substance de détection (Ac₂) qui est une immunoglobuline conjuguée à une substance fluorescente, et on vérifie si l'on détecte par fluorescence un produit de réaction (Ag₁-Ac₁-Ac₂) à une dilution de l'échantillon à tester inférieure ou égale à 1/200^{ème} , plus exactement égale à la dilution seuil utilisée pour la détection de l'antigène Ag₂, c'est à dire la plus petite dilution à partir de laquelle l'échantillon de sérum peut donner lieu à la détection d'une réaction contre les antigènes spécifiques Ag₂, cette dilution seuil étant variable en fonction du pathogène et de la méthode de détection et étant généralement comprise entre 1 :4 et 1 :200. Comme mentionné précédemment, pour chaque méthode de détection, on peut déterminer un titre en anticorps (Ac₂) donné, c'est à dire un seuil de dilution du sérum à tester donné, au delà duquel on ne détecte plus ledit anticorps (Ac₂) se liant à la protéine A.

Comme montré dans les exemples qui vont suivre dans le cas d'une méthode de détection par immunofluorescence, il est démontré que tout sérum humain incorporé dans une réaction d'immunofluorescence indirecte à une dilution inférieure ou égale à 1/200^{ème} présente une réaction contre *Staphylococcus aureus,* c'est à dire que le titre en anticorps anti*-Staphylococcus aureus* est d'au moins 1/200^{ème}.

Dans un mode de réalisation particulier, ledit agent microbien infectieux consistant dans ledit deuxième antigène est choisi parmi des micro-organismes comprenant une bactérie, un virus, un parasite ou un champignon.

Plus particulièrement, ledit deuxième antigène est une bactérie intracellulaire, et notamment, ledit deuxième antigène est choisi parmi les bactéries du genre *Rickettsia, Coxiella, Bartonella, Tropheryma, Ehrlichia, Chlamydia, Mycoplasma, Treponema, Borrelia,* et *Leptospira,* cette liste n'étant pas exhaustive.

Plus particulièrement encore, ledit deuxième antigène correspondant à une bactérie est responsable d'une endocardite.

Dans un autre mode de réalisation, ledit deuxième antigène est un antigène viral, notamment un virus choisi parmi les virus H.I.V., C.M.V. ou Epstein-Barr.

La présente invention a également pour objet une trousse de diagnostic utile pour la mise en oeuvre d'une méthode selon l'invention qui comprend au moins un contrôle positif de l'introduction d'un sérum humain dans l'échantillon à tester comprenant un dit premier antigène comprenant la protéine A et des réactifs permettant de détecter la présence d'un produit de réaction dudit premier antigène avec une immunoglobuline humaine.

Plus particulièrement, la trousse de diagnostic comprend :
- un support solide sur lequel on a déposé un dit premier antigène comprenant la protéine A et un dit deuxième antigène correspondant à un agent microbien infectieux à détecter, et une substance de détection d'un produit de réaction dudit premier antigène avec une immunoglobuline humaine comprenant une immunoglobuline anti-immunoglobuline humaine marquée qui ne réagit pas avec la protéine A, de préférence, une immunoglobuline anti-immunoglobuline humaine qui est une immunoglobuline de chèvre ou de poussin marquée avec un marquage fluorescent.

D'autres caractéristiques et avantages de la présente invention apparaîtront à la lumière des exemples qui vont suivre.

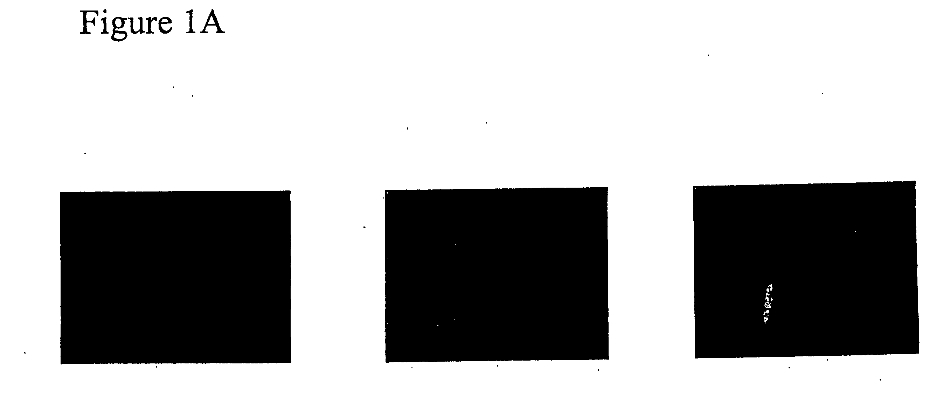

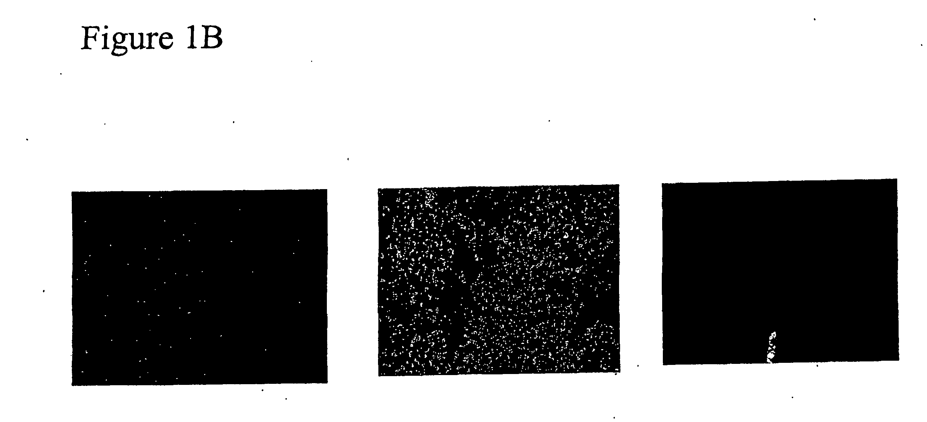

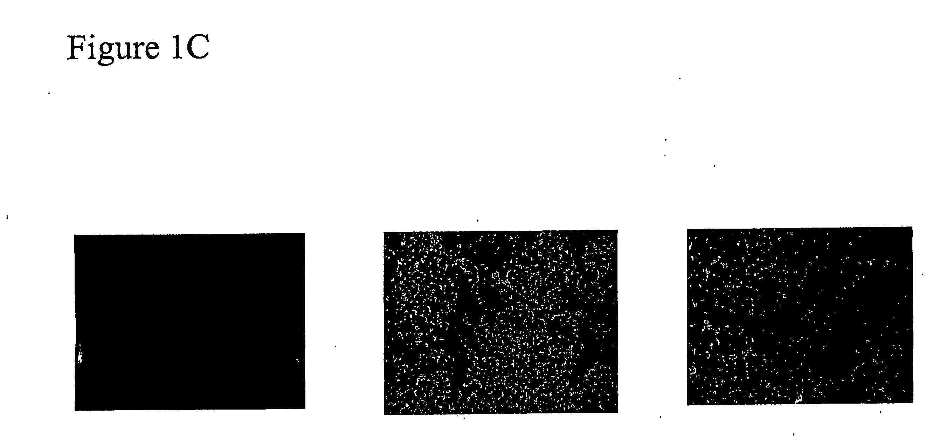

Les figures 1A, 1B et 1C représentent des tests de sérologie bactérienne réalisés par la méthode d'immunofluorescence indirecte illustrant l'invention.

### 1. Protocole de la méthode sérologique

### 1) Echantillons

Les échantillons testés comprennent :
- sérum de personne donneur de sang (n=100),
- sérum de patient présentant une endocardite (n=400),
- sérum de patient présentant la maladie des griffes du chat due à la bactérie *Bartonella henselae, et*
- sérum de patient présentant une infection aigüe à *Coxiella bumetti (n=50),*
- *sérum* de patient présentant des infections diverses (virus Epstein-Barr, infection V.I.H., infection à C.M.V.) (n=50)

Le sérum est décomplémenté par chauffage à 56°C pendant 30 minutes, il peut être conservé à 4°C si l'analyse est différée.

### 2) Matériel

- Lame *DINA TECH® 18 puits*
- Microplaque en U pour dilution *réf : Elvetec® 025085096*
- Microscope à fluorescence

### 3) Réactifs

- Témoin positif par antigène
- Colle Fluoprep *réf : Biomérieux 75521 conservation à 20°C*
- 10 aliquots d'antigènes: *Staphylococcus aureus ATCC 29213, Rickettsia conorii ATCC VR-141, Rickettsia slovaca, Rickettsia typhi ATCC VR-144, Coxiella bumetti Souche Mine Mile ATCC VR-616, Francisella tularensis A TCC 15482* Conservés à -30°C.
- *Bartonella henselae ATCC 49882, Bartonella quintana ATCC 49793, Bartonella clarridgeae* (souche déposée, collection Unité des Rickettsies, Faculté de médecine, Marseille), *Bartonella, massilae* (souche déposée, collection Unité des Rickettsies, Faculté de médecine, Marseille). Conservés à 4°C.
- Anticorps de *détection : immunoglobuline de chèvre anti-immunoglobuline humaine IGH* (Fluoline-H, bioMérieux sa, Marcy l'Etoile, France).

### 4) Préparation des réactifs

- Les lames doivent être dégraissées dans l'alcool méthylique 2 h minimum, idéalement toute la nuit.
- Préparation des antigènes

Après décongélation et mise à température ambiante, dilution au ½ dans du PBS stérile (conservation à 4°C). On s'assure au préalable de la qualité du lot d'antigène en le testant par un sérum témoin (c'est à dire à concentration d'anticorps connue) afin de déterminer la dilution optimale d'utilisation de cet antigène.

### 5) Préparation des lames

- On dépose les antigènes sur les spots des lames dégraissées à l'aide d'une plume différente pour chaque antigène. Ces plumes sont rincées, essuyées et séchées après chaque utilisation.
- La première lame de dépistage doit comprendre les antigènes suivants, toujours déposés dans la même position à partir du haut et dans le sens des aiguilles d'une montre : *Staphylococcus aureus, Coxiella burnetii, Rickettsia slovaca, Rickettsia conorii, Rickettsia typhi.* On fixe les lames 10 mn dans acétone.
- La deuxième lame de dépistage doit comprendre les antigènes suivants, toujours déposés dans la même position à partir du haut et dans le sens des aiguilles d'une montre : *Staphylococcus aureus, Bartonella henselae, Bartonella clarridgeae, Bartonella quintana, Francisella tularensis et B. massiliae au milieu.* Fixer les lames 10 mn dans l'alcool méthylique.
- *Rappel : la lecture au microscope sera inversée.*

### 6) Dilution des sérums à tester.

Les sérums doivent être décomplémentés, numérotés et enregistrés dans l'informatique.

### . Préparation des dilutions.

Trois dilutions 1/25, 1/50, 1/100 sont systématiquement réalisées dans du PBS lait (pour LCR dilution 1/2, 1/4, 1/8, pour piqûre de tiques 1/8)

### . Dépôt des dilutions :

- 30 µl de chacune des 3 dilutions de chaque patient les unes sous les autres.
- Le premier spot de la lame est réservé au témoin positif constitué par un pool de sérums positifs connu pour chacun des antigènes testés.
- Le dernier spot de la lame est réservé au témoin négatif (PBS lait).

On laisse incuber 30 minutes à 37°C en atmosphère humide.

### 7) Rinçage des lames

On lave les lames avec un jet de PBS et dans des bains de PBS tween, puis de l'eau distillée,

### 8) Dépôt des Immunoglobulines

Préparation : IgH fluorescente totale à diluer dans le PBS lait avec une goutte de bleu evans au 1/400 (sous réserve que la dilution ait été testée à l'ouverture du flacon selon la procédure développée au point n°4. On dépose 30µl sur chaque spot. On laisse incuber 30 minutes à 37°C en atmosphère humide puis on rince selon même procédure que décrite précédemment.

### 9) Montage des lames

. On dépose 3 gouttes de Fluoprep® sur les lames sèches. On dépose une lamelle sans la bouger et on laisse le Fluoprep® diffuser sur toute la lame. On met à l'abri de la lumière jusqu'à la lecture.

### 10) Lecture

Elle est réalisée sur le *microscope* à fluorescence. La lecture au microscope sera inversée par rapport au dépôt. On note toutes les positivités sur votre feuille de paillasse.

### 2. Résultats

Une série de sérum a été collectée chez des personnes donneurs de sang (témoins de la population générale) (n = 100) et chez des patients présentant une endocardite (n = 400), une maladie des griffes du chat due à la bactérie *Bartonella henselae* (n = 50), une infection aiguë à *Coxiella burnetii* (fièvre Q aigüe) (n = 50), ou des infections diverses caractérisées par une augmentation polyclonale, non-spécifique de taux des immunoglobulines sériques (infection à virus Epstein-Barr, infection à V.I.H., infection à C.M.V.) (n = 50).

Ce test sérologique a montré un titre d'anticorps anti-*Staphylococcus aureus 1* :400 chez 100% des patients présentant une endocardite, et un titre de 1 :200 chez 100% des autres patients ainsi que chez les donneurs de sang.

Une manipulation contrôle a été réalisée sur 10 tests dans lesquels l'échantillon à tester contenait uniquement l'immunoglobuline conjuguée de détection et dans lesquels le sérum de patient a été volontairement omis. Aucune fluorescence n'a été détectée sur ces 10 échantillons confirmant l'absence de réaction de l'immunoglobuline de chèvre avec la protéine A. Ces résultats démontrent que tout sérum humain incorporé dans une réaction d'immunofluorescence indirecte à une dilution ≤1 :200 présente une réaction contre Staphylococcus aureus ; et que cette réaction n'est pas altérée par les pathologies infectieuses, y compris les pathologies infectieuses induisant une hypergammaglubulinémie polyclonale et donc une modifiant fortement le taux et la qualité des immunoglobulines sériques humaines ; et que l'absence de sérum humain se traduit par une absence de fluorescence.

Pour les résultats présentés sur les figures 1A, 1B et 1C, le test a été réalisé contre trois antigènes, *S. aureus* ATCC 29 213 au centre de la lame, *R. conorii* ATCC VR-141 à gauche de la lame, et *B. henselae* ATCC 49882 à droite de la lame.
- figure 1A = sans un sérum humain,
- figure 1B = avec sérum humain positif pour *R. conorii*,
- figure 1C = avec sérum humain positif pour *B. henselae*.

L'omission de sérum humain entraîne une absence de fluorescence, (figure 1 A), l'introduction de sérum entraîne systématiquement une fluorescence contre *S. aureus* (figures 1B et 1C).

## Revendications

1. Méthode de diagnostic sérologique in-vitro dans laquelle on détecte la présence d'anticorps spécifiques d'un agent microbien infectieux dans un échantillon à tester, **caractérisée en ce que** l'on réalise les étapes suivantes dans lesquelles :
a) on dépose sur un support solide un premier antigène (Ag₁) comprenant une bactérie entière *Staphylococcus aureus* contenant la protéine A et au moins un deuxième antigène (Ag₂) qui est caractéristique d'un agent infectieux microbien (Ag₂), et
b) on fait réagir les dits premier (Ag₁) et second(s) (Ag₂) antigènes avec un échantillon à tester, et
c) on détecte si une immunoglobuline humaine (Ac₁) réagit avec ledit premier antigène (Ag₁) en faisant réagir le produit de la réaction (Ag₁-Ac₁) avec une substance de détection qui est une substance réagissant avec une immunoglobuline humaine et ne réagissant pas avec ledit premier antigène (Ag₁), et
d) on contrôle que ledit échantillon à tester contient un sérum humain en détectant si des immunoglobulines humaines réagissent avec ledit premier antigène.

2. Méthode de diagnostic sérologique selon la revendication 1, **caractérisé en ce que** ladite substance de détection est un anticorps secondaire de détection (Ac₂) qui est une immunoglobuline d'origine animale anti-immunoglobuline humaine marquée qui ne réagit pas avec la protéine A.

3. Méthode de diagnostic sérologique selon la revendication 2, **caractérisé en ce que** ladite immunoglobuline d'origine animale anti-immunoglobuline humaine (Ac₂) est une immunoglobuline de chèvre ou de poussin.

4. Méthode de diagnostic sérologique selon l'une des revendications 1 à 3, **caractérisé en ce que** ladite substance de détection est marquée par un marquage fluorescent.

5. Méthode de diagnostic sérologique selon la revendication 4, **caractérisé en ce que :**
- on réalise une série de tests à des dilutions de l'échantillon à tester croissantes et on met en oeuvre une dite substance de détection (Ac₂) qui est une immunoglobuline conjuguée à une substance fluorescente, et
- on vérifie si l'on détecte par fluorescence un produit de réaction (Ag₁-Ac₁-Ac₂) à une dilution de l'échantillon à tester inférieure ou égale à 1/200^{ème}.

6. Méthode de diagnostic sérologique selon l'une des revendication 1 à 5, **caractérisée en ce que** ledit agent microbien infectieux consistant dans ledit deuxième antigène est choisi parmi des micro-organismes comprenant une bactérie, un virus, un parasite ou un champignon.

7. Méthode de diagnostic sérologique selon la revendication 6, **caractérisée en ce que** ledit deuxième antigène (Ag₂) est une bactérie intracellulaire ou un virus.

8. Méthode de diagnostic sérologique selon la revendication 6 ou 7, **caractérisée en ce que** ledit deuxième antigène est choisi parmi les bactéries du genre *Rickettsia, Coxiella, Bartonella, Tropheryma, Ehrlichia, Chlamydia, Mycoplasma, Treponema, Borrelia,* et *Leptospira*.

9. Méthode de diagnostic sérologique selon la revendication 8, **caractérisée en ce que** ledit deuxième antigène correspondant à l'agent microbien infectieux est une bactérie responsable d'une endocardite.

10. Méthode de diagnostic sérologique selon l'une des revendications 7 ou 8, **caractérisée en ce que** ledit deuxième antigène correspondant audit agent microbien infectieux est un antigène viral choisi parmi les virus H.I.V., C.M.V. ou Epstein-Barr.

11. Trousse de diagnostic utile pour la mise en oeuvre d'une méthode selon l'une des revendications 1 à 10, **caractérisée en ce qu'**elle comprend au moins :
- un support solide sur lequel on a déposé au moins un dit deuxième antigène (Ag₂) correspondant à un agent microbien infectieux à détecter, et
- un contrôle positif de l'introduction d'un sérum humain dans l'échantillon à tester comprenant un dit premier antigène (Ag₁) comprenant une bactérie entière staphylococcus aureus contenant la protéine A, et
- des réactifs permettant de détecter la présence d'un produit de réaction dudit premier antigène (Ag₁) avec une immunoglobuline humaine (Ac₁), comprenant une dite substance de détection (Ac₂) comprenant une immunoglobuline anti immunoglobuline humaine marquée qui ne réagit pas avec la protéine A.

## Claims

1. *In vitro* serological diagnosis method in which, in a sample to be tested, the presence is detected of antibodies specific to an infectious microbial agent, **characterized in that** the following steps are performed, in which:
a) on a solid substrate are deposited a first antigen containing a whole *Staphylococcus aureus* bacterium containing protein A (Ag₁), and at least one second antigen (Ag₂) which is characteristic of a microbial infectious agent (Ag₂), and
b) the said first antigen (Ag₁) and second (Ag₂) antigen(s) are caused to react with a sample to be tested, and
c) it is detected whether a human immunoglobulin (Ac₁) reacts with said first antigen (Ag₁) by causing the reaction product (Ag₁-Ac₁) to react with a detection substance which is a substance reacting with a human immunoglobulin and not reacting with said first antigen (Ag₁), and
d) it is controlled that said sample to be tested contains a human serum by detecting whether human immunoglobulins react with said first antigen.

2. Serological diagnosis method as in claim 1, **characterized in that** the said detection substance is a secondary detection antibody (Ac₂) which is a labelled anti-human immunoglobulin which does not react with protein A.

3. Serological diagnosis method as in claim 2, **characterized in that** the said anti-human immunoglobulin (Ac₂) which is an immunoglobulin of animal origin, is goat or chick immunoglobulin.

4. Serological diagnosis method as in any of claims 1 to 3, **characterized in that** the said detection substance is labelled by fluorescent marking.

5. Serological diagnosis method as in claim 4, **characterized in that:**
- a series of tests is performed at increasing dilutions of the sample to be tested and the detection substance (Ac₂) is applied which is an immunoglobulin conjugated with a fluorescent substance, and
- it is verified whether a reaction product (Ag₁-Ac₁-Ac₂) can be detected by fluorescence at a dilution of the sample to be tested of 1/200 or less.

6. Serological diagnosis method as in any of claims 1 to 5, **characterized in that** said infectious microbial agent consisting of said second antigen is chosen from among micro-organisms containing a bacterium, a virus, a parasite or a fungus.

7. Serological diagnosis method as in claim 6, **characterized in that** said second antigen (Ag₂) is an intracellular bacterium or a virus.

8. Serological diagnosis method as in claim 6 or 7, **characterized in that** said second antigen is chosen from among bacteria of the genus *Rickettsia, Coxiella, Bartonella, Tropheryma, Ehrlichia, Chlamydia, Mycoplasma, Treponema, Borrelia,* and *Leptospira*.

9. Serological diagnosis method as in claim 8, **characterized in that** said second antigen corresponding to the infectious microbial agent is a bacterium responsible for endocarditis.

10. Serological diagnosis method as in either of claims 7 to 8, **characterized in that** said second antigen corresponding to said infectious microbial agent is a viral antigen chosen from among the H.I.V., C.M.V. or Epstein-Barr viruses.

11. Diagnosis kit which can be used to implement the method as in any of claims 1 to 10, **characterized in that** it includes at least:
- a solid substrate on which a said second antigen corresponding to an infectious microbial agent (Ag₂) to be detected has been deposited, and
- one positive control controlling inclusion of a human serum in the sample to be tested comprising a said first antigen containing a whole *Staphylococcus aureus* bacterium containing protein A (Ag₁), and
- reagents enabling the detection of the presence of a reaction product of said first antigen with a human immunoglobulin (Ac₁) comprising a said detection substance (Ac₂) comprising a labelled immunoglobulin which is an anti-human immunoglobulin which does not react with protein A.

## Patentansprüche

1. Serologisches in-vitro Diagnoseverfahren, bei dem man die Gegenwart von Antikörpern, die für ein infektiöses mikrobielles Reagenz spezifisch sind, in einer zu testenden Probe detektiert, **dadurch gekennzeichnet, daß** man die folgenden Schritte ausführt, in denen:
a) man auf einen festen Träger ein erstes Antigen (Ag₁) abscheidet, das ein vollständiges Bakterium *Staphylococcus aureus* umfaßt, das das Protein A enthält, und wenigstens ein zweites Antigen (Ag₂), das charakteristisch für ein infektiöses mikrobielles Antigen (Ag₂) ist, und
b) man die ersten (Ag₁) und zweiten (Ag₂) Antigene mit einer zu testenden Probe reagieren läßt und
c) man detektiert, ob ein menschliches Immunoglobulin (Ac₁) reagiert mit dem ersten Antigen (Ag₁), unter Reagierenlassen des Produkts der Reaktion (Ag₁-Ac₁) mit einer Detektionssubstanz, die eine Substanz ist, die mit einem Human-Immunoglobulin reagiert und nicht mit dem ersten Antigen (Ag₁) reagiert, und
d) man kontrolliert, daß die zu testende Probe ein menschliches Serum enthält, indem man detektiert, ob die Human-Immunoglobuline mit dem ersten Antigen reagieren.

2. Serologisches Diagnoseverfahren nach Anspruch 1, **dadurch gekennzeichnet, daß** die Detektionssubstanz ein sekundärer Detektionsantikörper (Ac₂) ist, der ein Immunoglobulin tierischen Ursprungs, nämlich ein markiertes Anti-Human-Immunoglobulin ist, das nicht mit dem Protein A reagiert hat.

3. Serologisches Diagnoseverfahren nach Anspruch 2, **dadurch gekennzeichnet, daß** das Immunoglobulin tierischen Ursprungs, nämlich ein Anti-Human-Immunoglobulin (Ac₂), ein Immunoglobulin aus Ziege oder Küken ist.

4. Serologisches Diagnoseverfahren nach einem der Ansprüche 1 bis 3, **dadurch gekennzeichnet, daß** die Detektionssubstanz mittels einer Fluoreszenzmarkierung markiert ist.

5. Serologisches Diagnoseverfahren nach Anspruch 4, **dadurch gekennzeichnet, daß:**
- man eine Testreihe mit ansteigenden Verdünnungen der zu testenden Probe ausführt und man eine genannte Detektionssubstanz (Ac₂) einsetzt, die ein Immunoglobulin ist, das an eine Fluoreszenzsubstanz konjugiert ist, und
- man überprüft, ob man durch Fluoreszenz ein Reaktionsprodukt (Ag₁-Ac₁-Ac₂) bei einer Verdünnung der zu testenden Probe unter oder gleich 1/200 detektiert.

6. Serologisches Diagnoseverfahren nach einem der Ansprüche 1 bis 5, **dadurch gekennzeichnet, daß** das infektiöse mikrobielle Reagenz, das aus dem zweiten Antigen besteht, gewählt ist unter Mikroorganismen, die ein Bakterium, einen Virus, einen Parasit oder einen Pilz umfassen.

7. Serologisches Diagnoseverfahren nach Anspruch 6, **dadurch gekennzeichnet, daß** das zweite Antigen (Ag₂) ein intrazelluläres Bakterium ist oder ein Virus.

8. Serologisches Diagnoseverfahren nach Anspruch 6 oder 7, **dadurch gekennzeichnet, daß** das zweite Antigen gewählt ist unter den Bakterien der Art *Rickettsia, Coxiella, Bartonella, Tropheryma, Ehrlichia, Chlamydia, Mycoplasma, Treponema, Borrella* und *Leptospira.*

9. Serologisches Diagnoseverfahren nach Anspruch 8, **dadurch gekennzeichnet, daß** das zweite Antigen, das dem infektiösen mikrobiellen Reagenz entspricht, ein Bakterium ist, das verantwortlich ist für eine Endokarditis.

10. Serologisches Diagnoseverfahren nach einem der Ansprüche 7 oder 8, **dadurch gekennzeichnet, daß** das zweite Antigen, das dem infektiösen mikrobiellen Reagenz entspricht, ein virales Antigen ist, das gewählt ist unter den Viren HIV, CMV oder Epstein-Barr.

11. Diagnosekit, verwendbar für die Ausführung eines Verfahrens nach einem der Ansprüche 1 bis 10, **dadurch gekennzeichnet, daß** er wenigstens umfaßt:
- einen festen Träger, auf den man wenigstens ein zweites genanntes Antigen (Ag₂) abgeschieden hat, das einem infektiösen zu detektierenden mikrobiellen Reagenz entspricht, und
- eine Positivkontrolle der Einführung eines menschlichen Serums in die zu testende Probe, umfassend ein erstes Antigen (Ag₁), das ein vollständiges Bakterium *Staphylococcus aureus* umfaßt, das Protein A enthält, und
- Reagenzien, die es erlauben, die Gegenwart eines Reaktionsprodukts des ersten Antigens (Ag₁) mit einem Human-Immunoglobulin (Ac₁) zu detektieren, umfassend eine genannte Detektionssubstanz (Ac₂), die ein Immunoglobulin, nämlich ein markiertes Anti-Human-Immunoglobulin, umfaßt, das nicht mit dem Protein A reagiert.
